# EUROPÄISCHE PATENTANMELDUNG

(11) **EP 1 561 444 A1**
(43) Veröffentlichungstag der Anmeldung: **10.08.2005**
(21) Anmeldenummer: 04005221.9
(22) Anmeldetag: 05.03.2004
(51) Int. Cl.: A61F 13/471

(54) **Inkontinenz-Einwegartikel für Manner**

(30) Priorität: 04.02.2004 PL 36478304; 17.02.2004 DE 20042526 U
(71) Anmelder: Torunskie Zaklady Materialow Opatrunkowych SA, PL-87-100 Torun (PL)
(72) Erfinder: Hoffmann, Tomasz, 87-100 Torun (PL); Kwiatkowski, Stanislaw, 87-300 Brodnica (PL)
(74) Vertreter: Tarvenkorn, Oliver

(57) **Zusammenfassung**

Die Erfindung betrifft einen hygienischen Harninkontinenz-Einwegartikel (10) für Männer, der mit wenigstens einer flüssigkeitsabsorbierenden Einlage versehen ist und im nicht benutzten Zustand eine in Draufsicht auf seine Flachseite etwa viereckige Außenkontur aufweist und sich etwa auf einer Hälfte seiner Länge, zwei seitliche Flügel bildend, zungenartig verjüngt.

Eine flüssigkeitsabsorbierende Einlage (3) ist zwischen einem flüssigkeitsundurchlässigen Backsheet (2) und einem dem Backsheet (2) abgewandten, flüssigkeitsdurchlässigen Topsheet (1) angeordnet.

Nach dem Zusammenfalten wird eine anatomisch anpassbare Absorptionstasche zur Aufnahme von Harntropfen des Patienten gebildet, die beim Gebrauch durch wenigstens ein Verschlusselement (4.1) vor dem Abrutschen vom Penis des Patienten gesichert ist.

## Beschreibung

Die Erfindung betrifft einen hygienischen Harninkontinenz -Einwegartikel für Männer, der mit einer flüssigkeitsabsorbierenden Einlage versehen ist und im nicht benutzten Zustand eine in Draufsicht auf seine Flachseite etwa viereckige Außenkontur aufweist und sich etwa auf einer Hälfte seiner Länge, zwei seitliche Flügel bildend zungenartig verjüngt.

Aus dem japanischen Abstrakt JP 07124190 A ist eine im nicht benutzten Zustand abgeflachte Tasche bekannt, in der eine flüssigkeitsabsorbierende, ebenso flache Einlage untergebracht ist. Die aus einem flüssigkeitsundurchlässigen Material bestehende Tasche hat in Draufsicht auf ihrer Flachseite einen etwa viereckigen Umriss, welcher sich etwa auf einer Hälfte der Taschenlänge zungenartig verjüngt. Nachteilig bei dem bekannten Produkt ist, dass dieses an der Unterhose fixiert werden muss. Das Produkt ist relativ dick und daher beeinträchtigt das Komfortgefühl.

Aufgabe der Erfindung ist, einen anatomisch anpassbaren, hygienischen Harninkontinenz-Einwegartikel für Männer zu konzipieren, bei dem keine Befestigung am Kleidungsstück erforderlich ist.

Diese Aufgabe ist durch einen Harninkontinenz-Einwegartikel der eingangs genannten Art gelöst, bei dem
- die flüssigkeitsabsorbierende Einlage zwischen einem flüssigkeitsundurchlässigen Backsheet und einem dem Backsheet abgewandten, flüssigkeitsdurchlässigen Topsheet angeordnet ist,
- und nach dem Zusammenfalten eine anatomisch anpassbare Absorptionstasche zur Aufnahme von Harntropfen vom Patienten bildet, die beim Gebrauch durch wenigstens ein Verschlusselement vor dem Abrutschen vom Körperteil des Patienten gesichert ist.

Der hygienische Harninkontinenz-Einwegartikel gemäß der Erfindung, im weiteren Einwegartikel genannt, eignet sich insbesondere für die Patienten mit Anfangssymptomen der Harninkontinenz, sogenannter Tropfinkontinenz.

Die anatomische Anpassbarkeit des Einwegartikels ist dadurch erreicht, dass sich die seitlichen Flügel und das verjüngte Teil in sehr einfacher Weise zusammenfalten, auf den Penis aufsetzen und dort mittels Verschlusselemente vor dem peinlichen Ausrutschen sichern lassen. Dies verbessert das Komfortgefühl des an Harninkontinenz leidenden Patienten wesentlich.

Die praktischen Versuche haben gezeigt, dass die anatomische Anpassbarkeit des Einwegartikels und dessen Handhabung durch Verwendung von Klettverschlüssen verbessert werden kann. Anstelle der Klettverschlüsse können andere Verschlusselemente, wie Klebebänder mit Schutzstreifen, zum Einsatz kommen. Es ist auch nicht ausgeschlossen, andere aus dem Textilbereich bekannten Verschlusselemente, wie Druckknöpfe, zu verwenden.

Vorzugsweise ist die Breite des flach liegenden, flüssigkeitsundurchlässigen Backsheets und des flüssigkeitsdurchlässigen Topsheets so bemessen, dass diese auf einer Höhe, die nicht kleiner als eine halbe Höhe des Einwegartikels ist, sich wenigstens um die Hälfte gegenüber dem verjüngten Teil vergrößert.

Vorzugsweise sind die beiden seitlichen Flügel zueinander spiegelsymmetrisch angeordnet. Abweichend von diesem Merkmal sind auch andere Konfigurationen, beispielsweise zwei asymmetrisch angeordnete oder ein einziger, verbreiteter Flügel denkbar.

Vorteilhaft ist, den Einwegartikel mit zwei längs der flüssigkeitsabsorbierenden Einlage verlaufenden Stegen auszustatten, die eine zusätzliche Sicherung gegen den seitlichen Auslauf von Flüssigkeit bilden.

Das flüssigkeitsdurchlässige Topsheet kann im allgemeinen aus Vliesmaterial, beispielsweise aus Polypropylen- oder Polyester-Vlies (PP; PES) bzw. aus einem Vlies auf Basis einer Polypropylen-Polyethylen-Mischung, oder aus perforierter Folie, wie Polypropylen,- Polyester- oder Polyethylenfolie hergestellt sein. Das Topsheet kann auch aus Textilgewebe oder papierartigem Material bestehen.

Das flüssigkeitsundurchlässige Backsheet kann aus einer Polyethylen-Isolationsfolie, einer atmungsaktiven Folie bzw. einer mit Vlies laminierten, atmungsaktiven oder nicht atmungsaktiven Folie bestehen. Das Backsheet kann auch aus einem dichten Gewebe, das mit einer wasserabweisenden, mikroporösen Beschichtung versehen ist, bestehen.

Die flüssigkeitsabsorbierende Einlage kann aus einer zerfaserten Cellulose, sogenannten Cellulosen-Pulpe, die vorzugsweise mit sogenanntem Superabsorber versetzt ist, bestehen.

Als Absorber kann auch ein flüssigkeitsabsorbierendes Band vom Typ "airlaid" verwendet sein.

Als flüssigkeitsabsorbierende Einlage kann auch eine Sachette mit flüssigkeitsabsorbierendem, pulver- oder granulatförmigen Absorber zum Einsatz kommen. Die Sachette kann aus unterschiedlichen Materialien, wie Textil, perforierte Folie oder papierartiges Material, bestehen.

Ausführungsbeispiele der Erfindung sind anhand der Zeichnung näher erläutert. Die Figuren zeigen:
- Fig. 1: einen Einwegartikel während des Zusammenfaltens, in einer perspektivischen Ansicht;
- Fig. 2: den Einwegartikel gemäß der Fig. 1 nach dem Zusammenfalten, ebenso in einer perspektivischen Ansicht;
- Fig. 3: den Einwegartikel gemäß der Fig. 1 in einer schematischen Draufsicht auf seine Flachseite;
- Fig. 4: einen Einwegartikel mit zwei Klettverschlüssen, in einer schematischen Draufsicht auf seine Flachseite;
- Fig. 5: einen Schnitt A-A gemäß der Fig. 4;
- Fig. 6: einen Einwegartikel mit drei Klettverschlüssen, in einer schematischen Draufsicht auf seine Flachseite;
- Fig. 7: einen schematischen Querschnitt durch den flachen Einwegartikel, in einer Exposionsdarstellung;
- Fig. 8: einen Einwegartikel mit einer zwei Klettverschlüssen und einer flüssigkeitsabsorbierenden Einlage von einem bogenförmigen Umriss, in einer schematischen Draufsicht auf seine Flachseite;
- Fig. 9: eine andere Ausführungsform des Einwegartikels, mit scharf endenden Flügeln, in einer schematischen Draufsicht auf seine Flachseite;
- Fig. 10: eine weitere Ausführungsform des Einwegartikels, mit asymmetrisch angeordneten Flügeln, in einer schematischen Draufsicht auf seine Flachseite; und
- Fig. 11: eine abweichende Ausführungsform des Einwegartikels, ebenso in einer schematischen Draufsicht auf seine Flachseite.

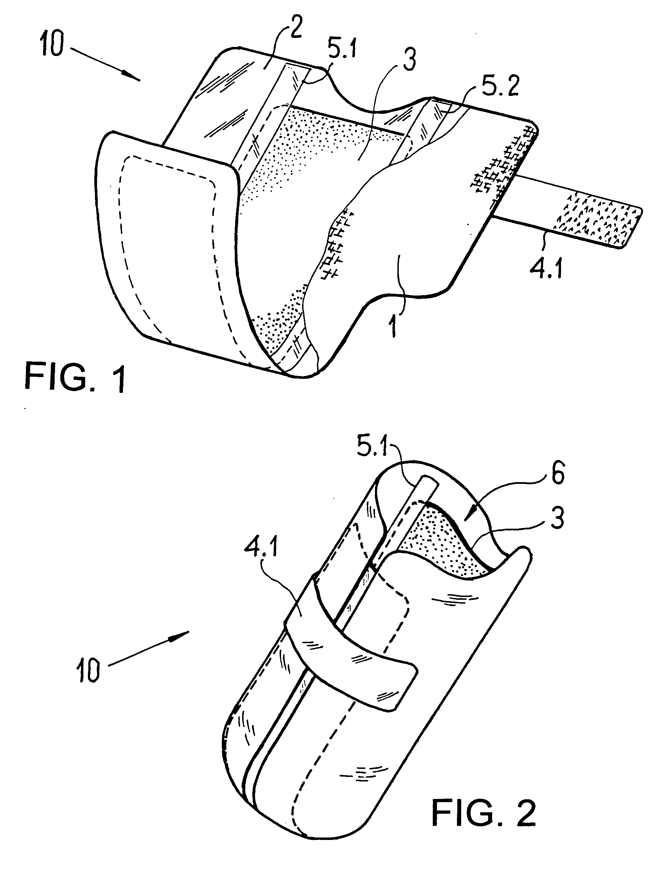

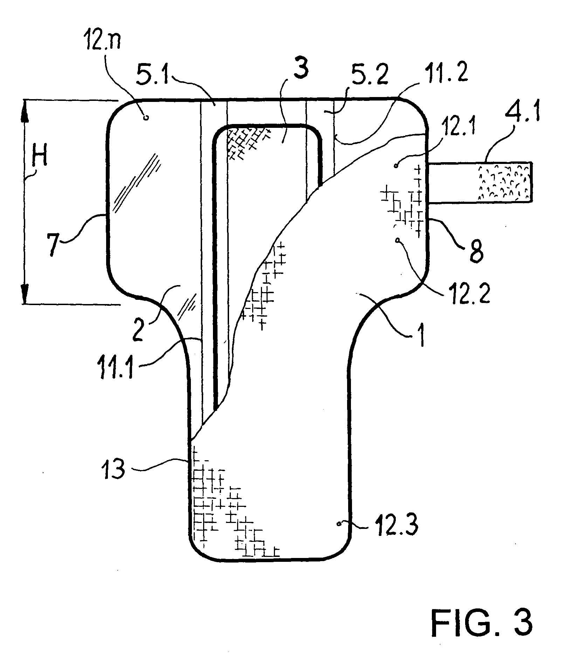

Die Figuren 1 und 2 zeigen einen Einwegartikel 10 während und nach dem Zusammenfalten eines in der Fig. 3 dargestellten Flachgebildes. Das Flachgebilde gemäß der Fig. 3 entspricht etwa einem zum Verkauf bestimmten Einwegartikel, der normalerweise in einer dichten, beutelartigen Verpackung (nicht dargestellt) untergebracht ist. Sowohl der Einwegartikel als auch die Verpackung kann sterilisiert sein und in diesen Form vertrieben werden.

Der Einwegartikel 10 setzt sich aus einem flüssigkeitsdurchlässigen Topsheet 1, einem deckungsgleichen flüssigkeitsundurchlässigen Backsheet 2, einer dazwischen untergebrachten flüssigkeitsabsorbierenden Einlage 3 und einem bandförmigen Klettverschluß 4.1 zusammen. Die flüssigkeitsabsorbierende Einlage 3 liegt zwischen zwei Stegen 5.1, 5.2, die im vorliegenden Fall mittels zwei länglicher Schweißnähte 11.1, 11.2 mit dem Topsheet 1 verbunden sind. Das Topsheet 1 und das Backsheet 2 sind mittels schematisch dargestellter Klebepunkte 12.1....12.n (vgl. Fig. 3), jedoch außerhalb des Bereiches zwischen den Stegen 5.1, 5.2 miteinander verbunden. Anstelle von Klebepunkten können Klebestreifen, Punktnähte etc. verwendet werden.

Es wird eine Konstruktion angestrebt, die sich nicht nur durch eine ausreichende Ansaugwirkung beim Harntröpfeln, sondern auch durch ein relativ kleines Volumen, auch beim Gebrauch, auszeichnet.

Darüber hinaus sind die zur Herstellung von Einwegartikeln verwendeten Materialien relativ dünn und biegsam. Das flüssigkeitsundurchlässige Backsheet 2 besteht aus einer mit Vlies laminierten, atmungsaktiven Folie. Das flüssigkeitsdurchlässige Topsheet 1 ist im vorliegenden Fall aus einem Polypropylen-Polyethylen-Vlies hergestellt. Die flüssigkeitsabsorbierende Einlage 3 besteht aus einem rechteckigen Bandabschnitt eines Absorbers vom Typ "airlaid" mit Superabsorber-Teilchen.

Das flüssigkeitsundurchlässige Backsheet 2 ist mit dem flüssigkeitsdurchlässigen Topsheet 1 deckungsgleich und weist zwei zueinander spiegelsymmetrisch angeordnete Flügel 7, 8 auf, die auf einer Höhe H, die etwa einer halben Höhe des gesamten Einwegartikels entspricht, in eine mittige, engere Zunge 13 übergehen.

Nach dem Zusammenfalten ergibt sich eine in der Fig. 2 gezeigte, anatomisch angepasste Absorptionstasche 6. Mit dem einzigen Klettverschluss 4.1 wird der Einwegartikel vor dem Ausrutschen vom Körperteil des Patienten gesichert. Die Verwendung des Klettverschlusses ermöglicht eine einfache und sichere Regulierung der Spannkraft.

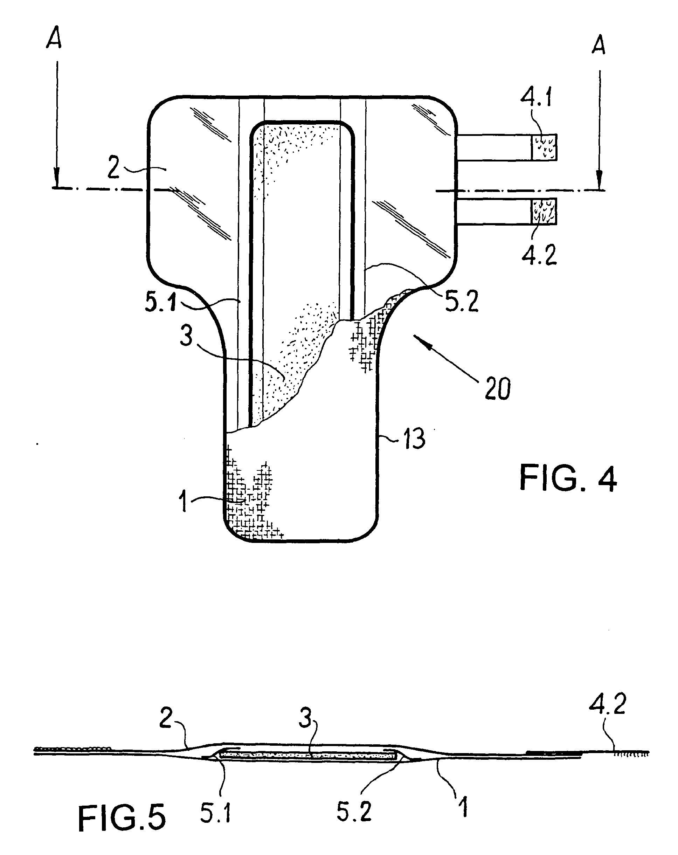

Ein in der Fig. 4 dargestellter Einwegartikel 20 unterscheidet sich von dem Einwegartikel 20 durch Anbringung von zwei Klettverschlüssen 4.1, 4.2. Der etwas vergrößerte Schnitt A-A zeigt Details des bandförmigen Klettverschlüsses (vgl. Fig.5), bei dem das Bandende mehrere Häkchen aufweist, die mit einer entsprechenden, am Teil der Oberfläche des Backsheets 2 angeordneten Vliesstruktur zusammenwirken.

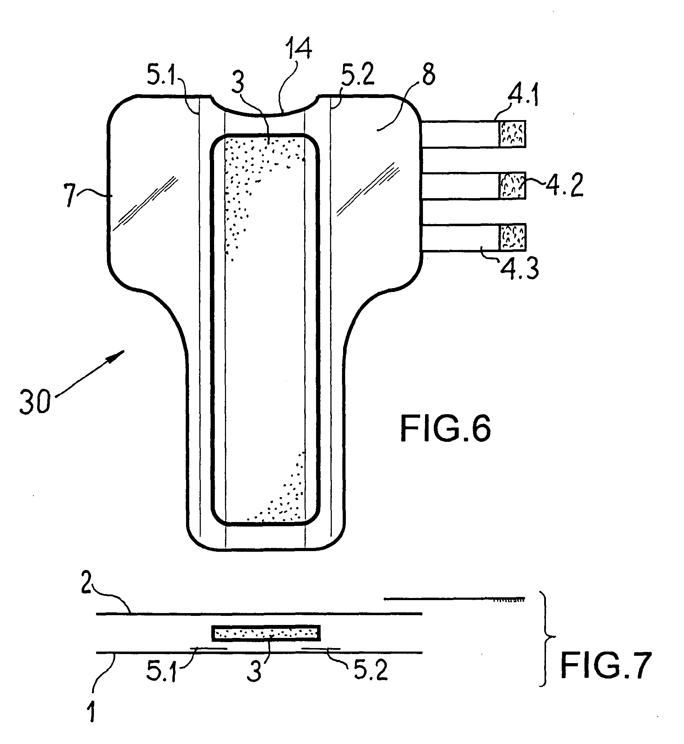

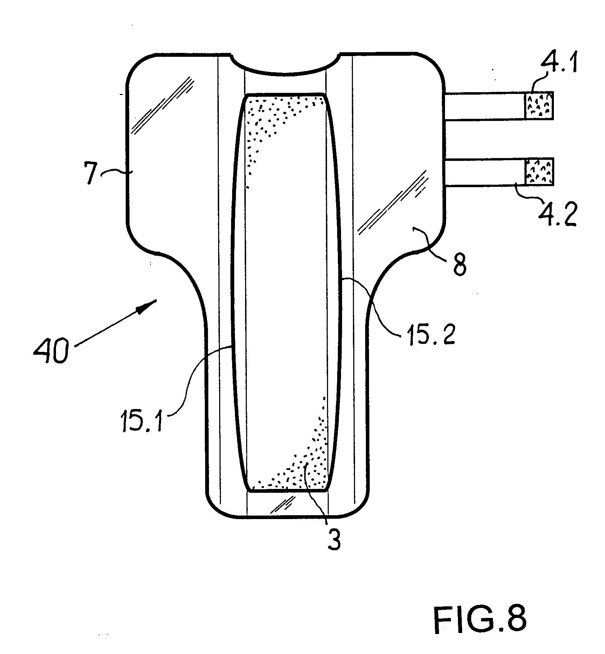

In den Figuren 6 und 7 ist schematisch ein Einwegartikel 30 mit drei Klettverschlüssen 4.1, 4.2, 4.3 dargestellt. Am oberen, mittleren Teil zwischen den Flügeln 7, 8 ist eine bogenförmige Aussparung 14 eingearbeitet.

Ein in der Fig. 8 gezeigter Einwegartikel 40 besitzt eine flüssigkeitsabsorbierende Einlage 3, deren beide Längskanten 15.1, 15.2 bogenförmig sind. Die Einlage 3 verjüngt sich an ihren beiden Enden.

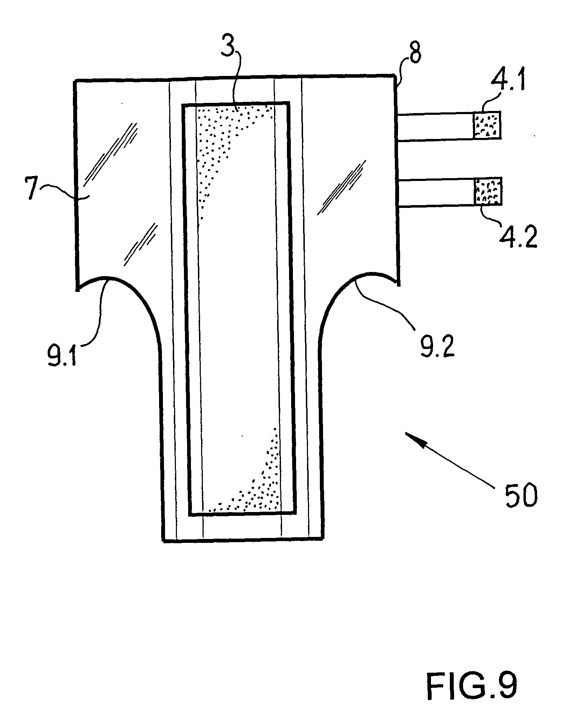

Bei einem in der Fig. 9 dargestellten Einwegartikel 50 sind zwei bogenartig verlaufende und zueinander spiegelsymmetrisch liegende Ausparungen 9.1, 9.2 zu sehen, die spitzenartig in die Seitenkanten der Flügel 7, 8 übergehen.

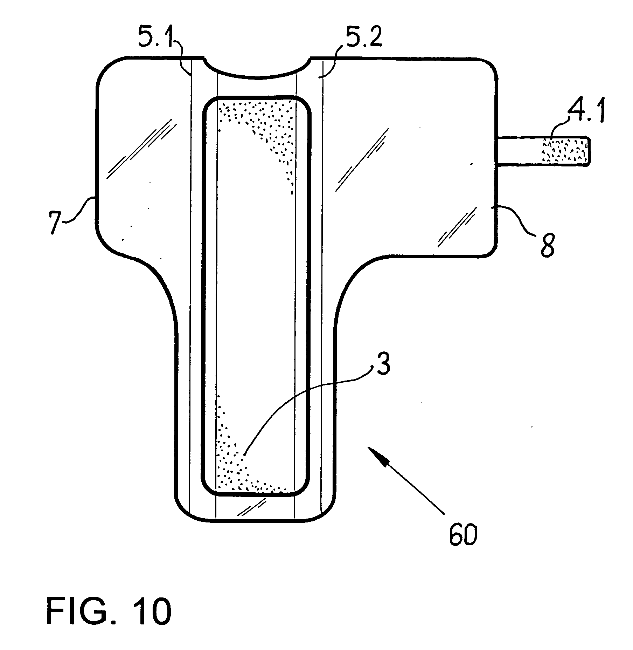

Bei einem in der Fig. 10 gezeigten Einwegartikel 50 handelt sich um eine asymmetrische Anordnung von Flügeln 7, 8. Der Flügel 8 ist wesentlich breiter als der Flügel 7.

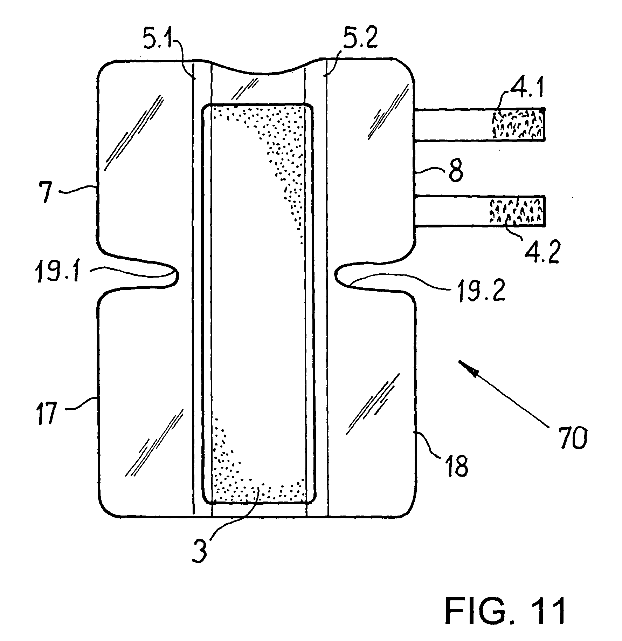

Eine abweichende, mögliche Ausführungsform (Bezugszahl 70) ist der Fig. 11 zu entnehmen. Der Einwegartikel 70 weist vier gleichförmige Flügel 7, 8, 17, 18 und zwei seitliche, tiefe Aussparungen 19.1, 19.2 auf, die die Flügel 7, 17 und 8, 18 voneinander trennen. Die Flügel können auch unterschiedlich breit sein. Die Klettverschlüsse können sowohl an oberen als auch an unteren Flügeln angeordnet sein.

## Patentansprüche

1. Hygienischer Harninkontinenz-Einwegartikel (10; 20; 30; 40; 50; 60; 70) für Männer, der mit wenigstens einer flüssigkeitsabsorbierenden Einlage versehen ist und im nicht benutzten Zustand eine in Draufsicht auf seine Flachseite etwa viereckige Außenkontur aufweist und sich etwa auf einer Hälfte seiner Länge, zwei seitliche Flügel bildend zungenartig verjüngt,
**dadurch gekennzeichnet, dass**
- die flüssigkeitsabsorbierende Einlage (3) zwischen einem flüssigkeitsundurchlässigen Backsheet (2) und einem dem Backsheet (2) abgewandten, flüssigkeitsdurchlässigen Topsheet (1) angeordnet ist,
- und dass nach dem Zusammenfalten eine anatomisch anpassbare Absorptionstasche (6) zur Aufnahme von Harntropfen vom Patienten bildet, die beim Gebrauch durch wenigstens ein Verschlusselement (4.1, 4.2, 4.3) vor dem Abrutschen vom Penis des Patienten gesichert ist.

2. Harninkontinenz-Einwegartikel nach Anspruch 1, **dadurch gekennzeichnet, dass** die Breite des flüssigkeitsundurchlässigen Backsheets (2) und des flüssigkeitsdurchlässigen Topsheets (1) auf einer Höhe (H), die nicht kleiner als eine halbe Höhe des hygienischen Einwegartikels ist, sich wenigstens um die Hälfte vergrößert.

3. Harninkontinenz-Einwegartikel nach Anspruch 1, **dadurch gekennzeichnet, dass** die seitlichen Flügel (7; 8) zueinander spiegelsymmetrisch angeordnet sind.

4. Harninkontinenz-Einwegartikel nach Anspruch 1, **dadurch gekennzeichnet, dass** dieser zwei längs der flüssigkeitsabsorbierenden Einlage (3) verlaufende Stege (5.1, 5.2) aufweist, die eine zusätzliche Sicherung gegen den seitlichen Auslauf von Flüssigkeit bilden.

5. Harninkontinenz-Einwegartikel nach Anspruch 1, **dadurch gekennzeichnet, dass** das Verschlusselement (4.1, 4.2, 4.3) ein Klettverschluss ist.

6. Harninkontinenz-Einwegartikel nach Anspruch 1, **dadurch gekennzeichnet, dass** das flüssigkeitsdurchlässige Topsheet (1) aus Vliesmaterial oder aus perforierter Folie hergestellt ist.

7. Harninkontinenz-Einwegartikel nach Anspruch 1, **dadurch gekennzeichnet, dass** das flüssigkeitsundurchlässige Backsheet (2) atmungsaktiv ist.
